(19) Europäisches Patentamt
European Patent Office
Office européen des brevets

(11) Publication number : **0 549 344 A1**

(12) # EUROPEAN PATENT APPLICATION

(21) Application number : **92311759.2**

(22) Date of filing : **23.12.92**

(51) Int. Cl.[5] : **C07D 251/16,** A01N 43/66, C07D 251/34

(30) Priority : **27.12.91 JP 358954/91**

(43) Date of publication of application : **30.06.93 Bulletin 93/26**

(84) Designated Contracting States : **DE ES FR GB IT**

(71) Applicant : **SUMITOMO CHEMICAL COMPANY, LIMITED**
**5-33, Kitahama 4-chome Chuo-ku**
**Osaka (JP)**

(72) Inventor : **Hiratsuka, Mitsunori**
**10-4-456, Sonehigashinocho-2-chome**
**Toyonaka-shi (JP)**
Inventor : **Uekawa, Toru**
**Mefuryo, 14-7, Mefu-2-chome**
**Takarazuka-shi (JP)**
Inventor : **Hirata, Naonori**
**61-7, Akashiadai-1-chome**
**Sanda-shi (JP)**
Inventor : **Saito, Kazuo**
**10-3-359, Sonehigashinocho-2-chome**
**Toyonaka-shi (JP)**
Inventor : **Shibata, Hideyuki**
**10-3-347, Sonehigashinocho-2-chome**
**Toyonaka-shi (JP)**

(74) Representative : **Coleiro, Raymond et al**
**MEWBURN ELLIS 2 Cursitor Street**
**London EC4A 1BQ (GB)**

(54) **Triazine derivatives as herbicides.**

(57) A herbicidally active triazine derivative has the formula :

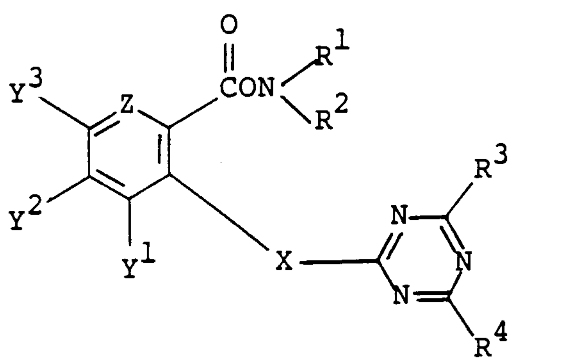

(I)

wherein each of $R^1$ and $R^2$, which may be the same or different, represents a hydrogen atom ; a $C_1$-$C_6$ alkyl group ; a $C_3$-$C_6$ alkenyl group ; a $C_3$-$C_6$ alkynyl group ; a halo-$C_1$-$C_6$ alkyl group ; a $C_1$-$C_6$ alkoxy-$C_1$-$C_6$ alkyl group ; a $C_3$-$C_6$ alkenyloxy-$C_1$-$C_6$ alkyl group ; a $C_3$-$C_6$ alkynyloxy-$C_1$-$C_6$ alkyl group ; a $C_1$-$C_6$ alkoxycarbonyl-$C_1$-$C_6$ alkyl group ; a $C_3$-$C_6$ alkenyloxycarbonyl-$C_1$-$C_6$ alkyl group ; a $C_3$-$C_6$ alkynyloxycarbonyl-$C_1$-$C_6$ alkyl group ; a cyano-$C_1$-$C_6$ alkyl group ; a cyclo-$C_3$-$C_8$ alkyl group ; a group represented by the formula,

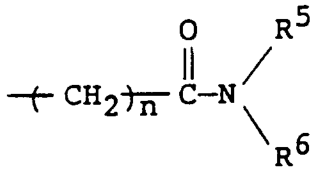

EP 0 549 344 A1

(in which each of $R^5$ and $R^6$, which may be the same or different, represents a hydrogen atom, a $C_1$-$C_6$ alkyl group, a $C_3$-$C_6$ alkenyl group or a $C_3$-$C_6$ alkynyl group, and n represents an integer of 1, 2, 3 or 4) ; a group represented by the formula,

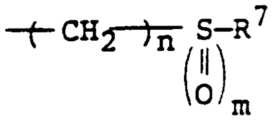

(in which n is as defined above, $R^7$ represents a $C_1$-$C_6$ alkyl group, a $C_3$-$C_6$ alkenyl group or a $C_3$-$C_6$ alkynyl group, and m represents an integer of 0, 1 or 2) ; a phenyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms ; or a benzyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms, or $R^1$ and $R^2$ are bonded together at their ends to form a $C_4$-$C_7$ alkylene group optionally substituted with at least one member selected from the group consisting of $C_1$-$C_6$ alkyl groups and halogen atoms or $R^1$ and $R^2$ are bonded together at their ends to form a $C_3$-$C_6$ alkylene group containing a hetero atom optionally substituted with at least one member selected from the group consisting of $C_1$-$C_6$ alkyl groups and halogen atoms (the hetero atom in $R^1$ and $R^2$ means a nitrogen atom, an oxygen atom or a sulfur atom),

each of $R^3$ and $R^4$, which may be the same or different, represents a $C_1$-$C_6$ alkyl group ; a $C_1$-$C_6$ alkoxy group ; a halo-$C_1$-$C_6$ alkoxy group ; or a halogen atom,

X represents an oxygen atom or a sulfur atom,

Z represents CH, N or $CY^4$,

each of $Y^1$, $Y^2$ and $Y^3$, which may be the same or different, represents a hydrogen atom ; a halogen atom ; a $C_1$-$C_6$ alkyl group ; a $C_1$-$C_6$ alkoxy group ; a nitro group ; a cyano group ; or a phenyl group, and

$Y^4$ represents a hydroxy group ; a mercapto group ; a nitro group ; a halogen atom ; a $C_1$-$C_6$ alkyl group ; a $C_2$-$C_6$ alkenyl group ; a $C_2$-$C_6$ alkynyl group ; a $C_1$-$C_6$ alkoxy group ; a $C_3$-$C_6$ alkenyloxy group ; a $C_3$-$C_6$ alkynyloxy group ; a halo-$C_1$-$C_6$ alkyl group ; a halo-$C_2$-$C_6$ alkenyl group ; a halo-$C_2$-$C_6$ alkynyl group ; a halo-$C_1$-$C_6$ alkoxy group ; a halo-$C_3$-$C_6$ alkenyloxy group ; a halo-$C_3$-$C_6$ alkynyloxy group ; a $C_1$-$C_6$ alkoxy-$C_1$-$C_6$ alkyl group ; a $C_3$-$C_6$ alkenyloxy-$C_1$-$C_6$ alkyl group ; a $C_3$-$C_6$ alkynyloxy-$C_1$-$C_6$ alkyl group ; a cyano group ; a formyl group ; a carboxyl group ; a ($C_1$-$C_6$ alkoxy)carbonyl group ; a ($C_3$-$C_6$ alkenyloxy)carbonyl group ; a ($C_3$-$C_6$ alkynyloxy)carbonyl group ; a phenyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms ; a phenoxy group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms ; a phenylthio group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro, cyano groups and halogen atoms ; a benzyloxy group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms ; a benzylthio group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms, a group represented by the formula,

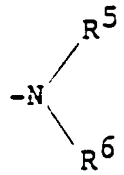

(in which each of $R^5$ and $R^6$ as described above) ; a group represented by the formula,

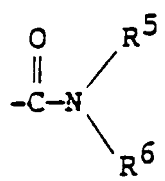

(in which $R^5$ and $R^6$ are as defined above) ; a group represented by the formula,

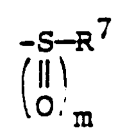

(in which $R^7$ and m are as described above) ; a group represented by the formula



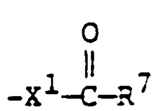

(in which $R^7$ is as defined above and $X^1$ is an oxygen atom or a sulfuratom or a group represented by the

(in which each of $R^5$ and $R^6$, which may be the same or different, represents a hydrogen atom, a $C_1$-$C_6$ alkyl group, a $C_3$-$C_6$ alkenyl group or a $C_3$-$C_6$ alkynyl group, and n represents an integer of 1, 2, 3 or 4); a group represented by the formula,

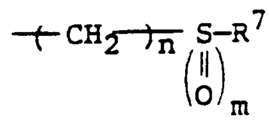

(in which n is as defined above, $R^7$ represents a $C_1$-$C_6$ alkyl group, a $C_3$-$C_6$ alkenyl group or a $C_3$-$C_6$ alkynyl group, and m represents an integer of 0, 1 or 2); a phenyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; or a benzyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms, or $R^1$ and $R^2$ are bonded together at their ends to form a $C_4$-$C_7$ alkylene group optionally substituted with at least one member selected from the group consisting of $C_1$-$C_6$ alkyl groups and halogen atoms or $R^1$ and $R^2$ are bonded together at their ends to form a $C_3$-$C_6$ alkylene group containing a hetero atom optionally substituted with at least one member selected from the group consisting of $C_1$-$C_6$ alkyl groups and halogen atoms (the hetero atom in $R^1$ and $R^2$ means a nitrogen atom, an oxygen atom or a sulfur atom),

each of $R^3$ and $R^4$, which may be the same or different, represents a $C_1$-$C_6$ alkyl group; a $C_1$-$C_6$ alkoxy group; a halo-$C_1$-$C_6$ alkoxy group; or a halogen atom,

X represents an oxygen atom or a sulfur atom,

Z represents CH, N or $CY^4$,

each of $Y^1$, $Y^2$ and $Y^3$, which may be the same or different, represents a hydrogen atom; a halogen atom; a $C_1$-$C_6$ alkyl group; a $C_1$-$C_6$ alkoxy group; a nitro group; a cyano group; or a phenyl group, and

$Y^4$ represents a hydroxy group; a mercapto group; a nitro group; a halogen atom; a $C_1$-$C_6$ alkyl group; a $C_2$-$C_6$ alkenyl group; a $C_2$-$C_6$ alkynyl group; a $C_1$-$C_6$ alkoxy group; a $C_3$-$C_6$ alkenyloxy group; a $C_3$-$C_6$ alkynyloxy group; a halo-$C_1$-$C_6$ alkyl group; a halo-$C_2$-$C_6$ alkenyl group; a halo-$C_2$-$C_6$ alkynyl group; a halo-$C_1$-$C_6$ alkoxy group; a halo-$C_3$-$C_6$ alkenyloxy group; a halo-$C_3$-$C_6$ alkynyloxy group; a $C_1$-$C_6$ alkoxy-$C_1$-$C_6$ alkyl group; a $C_3$-$C_6$ alkenyloxy-$C_1$-$C_6$ alkyl group; a $C_3$-$C_6$ alkynyloxy-$C_1$-$C_6$ alkyl group; a cyano group; a formyl group; a carboxyl group; a ($C_1$-$C_6$ alkoxy)carbonyl group; a ($C_3$-$C_6$ alkenyloxy)carbonyl group; a ($C_3$-$C_6$ alkynyloxy)carbonyl group; a phenyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a phenoxy group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a phenylthio group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro, cyano groups and halogen atoms; a benzyloxy group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a benzylthio group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms, a group represented by the formula,

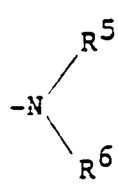

(in which each of $R^5$ and $R^6$ as described above); a group represented by the formula,

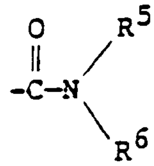

(in which $R^5$ and $R^6$ are as defined above); a group represented by the formula,

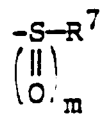

(in which $R^7$ and m are as described above); a group represented by the formula

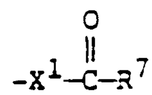

(in which $R^7$ is as defined above and $X^1$ is an oxygen atom or a sulfuratom or a group represented by the formula,

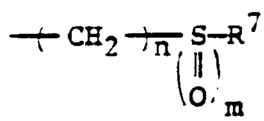

(in which $R^7$, m and n are as defined above). A process for producing the derivative and a herbicidal composition containing the same as an active ingredient are described.

The present invention relates to a novel triazine derivative, a method for producing the same and their use as herbicides.

Japanese Patent Application KOKAI No. 63-258467etc. disclose that triazine derivatives can be used as an active ingredient for herbicides.

However, these compounds are not always said to be satisfactory because they are insufficient as herbicides.

On the other hand, a large number of herbicides for crop lands or non-crop lands are now in use. However, there are many kinds of weed to be controlled and generation of the weeds extends over a long period of time, so that development of herbicides having a higher herbicidal effect and a broader herbicidal spectrum is being desired. Further, in recent years, no-till cultivation is carried out for the purposes of saving in labor, extension of cultivation period, prevention of soil running-off, etc. Because of this, it is being much desired to develop herbicides having both a high post-emergence herbicidal activity against weeds and a pre-emergence herbicidal activity excellent in residual effect, and besides having excellent selectivity of crops when crops are cultivated after application of herbicides.

In view of these circumstances, the present inventors have extensively studied, and as a result, have found that a triazine derivative represented by the following formula (I) is an excellent compound as a herbicide which can control weeds widely generated in crop lands or non-crop lands at low dosage rate, has a broad herbicidal spectrum and also can safely be used for no-till cultivation. The present invention is based on this finding.

The present invention provides a triazine derivative represented by the formula (I) [hereinafter referred to as present compound(s)], a method for producing the same and their use as herbicides;

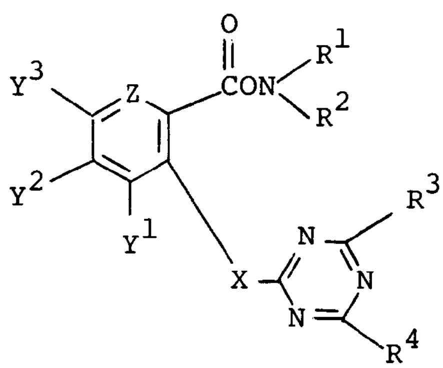

(I)

wherein each of $R^1$ and $R^2$, which may be the same or different, represents a hydrogen atom; a $C_1$-$C_6$ alkyl group; a $C_3$-$C_6$ alkenyl group; a $C_3$-$C_6$ alkynyl group; a halo-$C_1$-$C_6$ alkyl group; a $C_1$-$C_6$ alkoxy-$C_1$-$C_6$ alkyl group; a $C_3$-$C_6$ alkenyloxy-$C_1$-$C_6$ alkyl group; a $C_3$-$C_6$ alkynyloxy-$C_1$-$C_6$ alkyl group; a $C_1$-$C_6$ alkoxycarbonyl-$C_1$-$C_6$ alkyl group; a $C_3$-$C_6$ alkenyloxycarbonyl-$C_1$-$C_6$ alkyl group; a $C_3$-$C_6$ alkynyloxycarbonyl-$C_1$-$C_6$ alkyl group; a cyano-$C_1$-$C_6$ alkyl group; a cyclo-$C_3$-$C_6$ alkyl group; a group represented by the formula

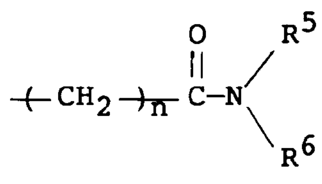

(in which each of $R^5$ and $R^6$, which may be the same or different, represents a hydrogen atom, a $C_1$-$C_6$ alkyl group, a $C_3$-$C_6$ alkenyl group or a $C_3$-$C_6$ alkynyl group, and n represents an integer of 1, 2, 3 or 4); a group represented by the formula,

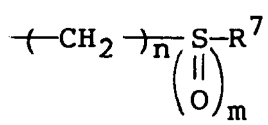

(in which n is as defined above, $R^7$ represents a $C_1$-$C_6$ alkyl group, a $C_3$-$C_6$ alkenyl group or a $C_3$-$C_6$ alkynyl group, and m represents an integer of 0, 1 or 2); a phenyl group optionally substituted with at least one member

selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; or a benzyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms, or $R^1$ and $R^2$ are bonded together at their ends to form a $C_4$-$C_7$ alkylene group optionally substituted with at least one member selected from the group consisting of $C_1$-$C_6$ alkyl groups and halogen atoms, or $R^1$ and $R^2$ are bonded together at their ends to form a $C_3$-$C_6$ alkylene group containing a hetero atom optionally substituted with at least one member selected from the group consisting of $C_1$-$C_6$ alkyl groups and halogen atoms (the hetero atom in $R^1$ and $R^2$ means a nitrogen atom, an oxygen atom or a sulfur atom),

each of $R^3$ and $R^4$, which may be the same or different, represents a $C_1$-$C_6$ alkyl group; a $C_1$-$C_6$ alkoxy group; a halo-$C_1$-$C_6$ alkoxy group; or a halogen atom,

X represents an oxygen atom or a sulfur atom,

Z represents CH, N or $CY^4$,

each of $Y^1$, $Y^2$ and $Y^3$, which may be the same or different, represents a hydrogen atom; a halogen atom; a $C_1$-$C_6$ alkyl group; a $C_1$-$C_6$ alkoxy group; a nitro group; a cyano group; or a phenyl group, and

$Y^4$ represents a hydroxyl group; a mercapto group; a nitro group; a halogen atom; a $C_1$-$C_6$ alkyl group; a $C_2$-$C_6$ alkenyl group; a $C_2$-$C_6$ alkynyl group; a $C_1$-$C_6$ alkoxy group; a $C_3$-$C_6$ alkenyloxy group; a $C_3$-$C_6$ alkynyloxy group; a halo-$C_1$-$C_6$ alkyl group; a halo-$C_2$-$C_6$ alkenyl group; a halo-$C_2$-$C_6$ alkynyl group; a halo-$C_1$-$C_6$ alkoxy group; a halo-$C_3$-$C_6$ alkenyloxy group; a halo-$C_3$-$C_6$ alkynyloxy group; a $C_1$-$C_6$ alkoxy-$C_1$-$C_6$ alkyl group; a $C_3$-$C_6$ alkenyloxy-$C_1$-$C_6$ alkyl group; a $C_3$-$C_6$ alkynyloxy-$C_1$-$C_6$ alkyl group; a cyano group; a formyl group; a carboxyl group; a ($C_1$-$C_6$ alkoxy)carbonyl group; a ($C_3$-$C_6$ alkenyloxy)carbonyl group; a ($C_3$-$C_6$ alkynyloxy)carbonyl group; a phenyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a phenoxy group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a phenylthio group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a benzyloxy group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a benzylthio group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a group represented by the formula,

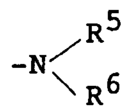

(in which each of $R^5$ and $R^6$ are as described above);
a group represented by the formula

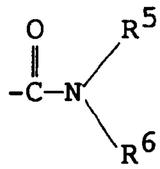

(in which $R^5$ and $R^6$ are as defined above); a group represented by the formula

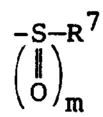

(in which $R^7$ and m are as described above); a group represented by the formula,

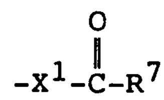

(in which $R^7$ is as defined above and $X^1$ is an oxygen atom or a sulfur atom), or a group representd by the formula,

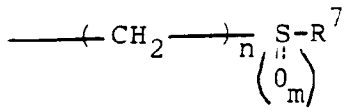

(in which $R^7$ and m are as defined above and n is as described above). In another aspect $R^1$ and $R^2$ are as defined in accompanying Claim 2. In the compound of the formula (I), each of the substituents $R^3$ and $R^4$, which may be the same or different, is preferably a $C_1$-$C_6$ alkoxy group, and more preferably, both of them are methoxy groups. X is preferably an oxygen atom. Z is preferably N or $CY^4$ in which $Y^4$ represents a halogen atom or a phenyl group. More preferably Z is $CY^4$ wherein $Y^4$ is a phenyl group. Each of $Y^1$, $Y^2$ and $Y^3$ is preferably a hydrogen atom or a fluorine atom.

Specific examples of the pyrimidine derivative of the present invention include

4,6-dimethoxy-2-{3-phenyl-2-(N,N-dimethylaminooxycarbonyl)phenoxy}-1,3,5-triazine,

4,6-dimethoxy-2-{3-fluoro-2-(N,N-dimethylaminooxycarbonyl)phenoxy}-1,3,5-triazine,

4,6-dimethoxy-2-{3-chloro-2-(N,N-dimethylaminooxycarbonyl)phenoxy}-1,3,5-triazine.

In the formula [I], $R^1$, $R^2$, $R^5$, $R^6$ and $R^7$ represent alkyl groups, such as methyl, ethyl, n-propyl, i-propyl, n-butyl, sec-butyl, tert-butyl and n-hexyl groups and the like; alkenyl groups having 3 or more carbon atoms, such as allyl, 1-butenyl, 2-butenyl, 3-butenyl, 2-pentenyl, 3-pentenyl and 2-hexenyl groups and the like; or alkynyl groups having 3 or more carbon atoms, such as propargyl, 1-butynyl, 2-butynyl, 2-pentynyl, 3-pentynyl and 2-hexynyl groups and the like. Moreover, $R^1$ and $R^2$ represent haloalkyl groups, such as 2-chloroethyl, 2-bromoethyl, 3-fluoropropyl, 2-chloropropyl, 4-chlorobutyl and 6-chlorohexyl groups and the like; alkoxyalkyl groups, such as methoxymethyl, ethoxymethyl, 2-methoxyethyl, 4-n-propoxybutyl, 2-n-butoxyethyl and 6-hexyloxyhexyl groups and the like; alkenyloxyalkyl groups of which alkenyloxy moieties have 3 or more carbon atoms, such as allyloxymethyl, 2-allyloxyethyl, 4-allyloxybutyl, 3-(2-butenyloxy)propyl and 6-(2-hexenyloxy)hexyl groups and the like; alkynyloxyalkyl groups of which alkynyloxy moieties have 3 or more carbon atoms, such as propargyloxymethyl, 2-propargyloxyethyl, 4-propargyloxybutyl, 3-(2-butynyloxy)propyl and 6-(2-hexynyloxy)hexyl groups and the like; alkoxycarbonylalkyl groups, such as methoxy-carbonylmethyl, ethoxycarbonylmethyl, 1-methoxycarbonylethyl, 2-methoxycarbonylethyl, 3-ethoxycarbonylpropyl, 4-n-butoxycarbonylbutyl and 6-n-hexyloxycarbonylhexyl groups and the like; alkenyloxycarbonylalkyl groups of which alkenyloxy moieties have 3 or more carbon atoms, such as allyloxycarbonylmethyl, 2-allyloxycarbonylethyl, 2-(2-butenyloxycarbonyl)ethyl, 3-(3-butenyloxycarbonyl)propyl, 4-(2-butenyloxycarbonyl)butyl and 6-(2-hexenyloxycarbonyl)hexyl groups and the like; alkynyloxycarbonylalkyl groups of which alkynyloxy moieties have 3 or more carbon atoms, such as propargyloxycarbonylmethyl, 2-propargyloxycarbonylethyl, 2-(2-butynyloxycarbonyl)ethyl, 3-(3-butynyloxycarbonyl)propyl, 4-(2-butynyloxycarbonyl)butyl and 6-(2-hexynyloxycarbonyl)hexyl groups and the like; cyanoalkyl groups, such as cyanomethyl, 2-cyanoethyl, 3-cyanopropyl, 4-cyanobutyl and 6-cyanohexyl groups and the like; or cycloalkyl groups, such as cyclopropyl, cyclobutyl, cyclopentyl, cyclohexyl and cycloheptyl groups and the like;

$R^3$ and $R^4$ represent alkyl groups, such as methyl, ethyl, n-propyl, i-propyl, n-butyl, sec-butyl, tert-butyl and n-hexyl groups and the like; alkoxy groups, such as methoxy, ethoxy, n-propoxy, i-propoxy, n-butoxy and n-hexyloxy groups and the like; or haloalkoxy groups, such as monofluoromethoxy, difluoromethoxy, trifluoromethoxy and 2,2,2-trifluoroethoxy groups and the like;

$Y^1$, $Y^2$, $Y^3$ and $Y^4$ represent alkyl groups, such as methyl, ethyl, n-propyl, i-propyl, n-butyl, sec-butyl, tert-butyl and n-hexyl groups and the like; or alkoxy groups, such as methoxy, ethoxy, n-propoxy, i-propoxy, n-butoxy and n-hexyloxy groups and the like;

$Y^4$ represents an alkenyl group, such as vinyl, allyl, 1-butenyl, 2-butenyl, 3-butenyl, 2-pentenyl, 3-pentenyl and 2-hexenyl groups and the like; an alkynyl group, such as ethynyl, propargyl, 1-butynyl, 2-butynyl, 2-pentynyl, 3-pentynyl and 2-hexynyl groups and the like; an alkenyloxy group having 3 or more carbon atoms, such as allyloxy, 2-butenyloxy, 3-butenyloxy and 2-hexenyloxy groups and the like; an alkynyloxy group having 3 or more carbon atoms, such as propargyloxy, 2-butynyloxy, 3-butynyloxy and 2-hexynyloxy groups and the like; a haloalkyl group, such as fluoromethyl, difluoromethyl, trifluoromethyl, 2-chloroethyl and

3-bromopropyl groups and the like; a haloalkenyl group, such as 1-chlorovinyl, 3-chloroallyl, 5-bromo-2-pentenyl, 6-iodo-2-hexenyl and 5,5,5-trifluoro- 2-pentenyl groups and the like; a haloalkynyl group, such as 2-iodoethynyl, 5-bromo-2-pentynyl, 6-iodo-2-hexynyl and 5,5,5-trifluoro-2-pentynyl groups and the like; a haloalkoxy group, such as fluoromethoxy, difluoromethoxy, trifluoromethoxy and 1,1,2,2-tetrafluoroethoxy groups and the like; a haloalkenyloxy group having 3 or more cabon atoms, such as 3-chloroallyloxy, 5-bromo-2-pentenyloxy, 6-iodo-2-hexenyloxy and 5,5,5-trifluoro-2-pentenyloxy groups and the like; a haloalkynyloxy group having 3 or more carbon atoms, such as 5-bromo-2-pentynyloxy, 5-chloro-2-pentynyloxy, 6-iodo-2-hexenyloxy, 5,5,5-trifluoro-2-pentynyloxy and 3-iodopropargyloxy groups and the like; an alkoxyalkyl group, such as methoxymethyl, ethoxymethyl, 2-methoxyethyl, 4-n-propoxybutyl, 2-n-butoxyethyl and 6-hexyloxyhexyl groups and the like; an alkenyloxyalkyl group of which alkenyloxy moiety has 3 or more carbon atoms, such as allyloxymethyl, 2-allyloxyethyl, 4-allyloxybutyl, 3-(2-butenyloxy)propyl and 6-(2-hexenyloxy)hexyl groups and the like; an alkynyloxyalkyl group of which alkynyloxy moiety has 3 or more carbon atoms, such as propargyloxymethyl, 2-propargyloxyethyl, 4-propargyloxybutyl, 3-(2-butynyloxy)propyl and 6-(2-hexynyloxy)hexyl groups and the like; an alkoxycarbonyl group, such as methoxycarbonyl, ethoxycarbonyl, n-butoxycarbonyl and 6-hexyloxycarbonyl groups and the like; an alkenyloxycarbonyl group of which alkenyloxy moiety has 3 or more carbon atoms, such as allyloxycarbonyl, 2-butenyloxycarbonyl, 3-butenyloxycarbonyl and 2-hexenyloxycarbonyl groups and the like; or an alkynyloxycarbonyl group of which alkynyloxy moiety has 3 or more carbon atoms, such as propargyloxycarbonyl, 2-butynyloxycarbonyl, 3-butynyloxycarbonyl and 2-hexynyloxycarbonyl groups and the like;

a halogen atom represents fluorine, chlorine, bromine or iodine atom. When $R^1$ and $R^2$ represent phenyl or benzyl groups, which may be substituted with R (wherein R is a $C_1$-$C_6$ alkyl, $C_1$-$C_6$ alkoxy, $C_1$-$C_6$ haloalkyl, ($C_1$-$C_6$ alkoxy)carbonyl, nitro, cyano group or a halogen atom) or $R^1$ and $R^2$ are bonded together at their ends to form an alkylene group or an alkylene group having one or more hetero atoms, which may be substituted with P (wherein P is a $C_1$-$C_6$ alkyl group or a halogen atom), or $Y^4$ represents a phenyl, phenoxy, phenylthio, benzyloxy or benzylthio group, which may be substituted with R (wherein R is a $C_1$-$C_6$ alkyl, $C_1$-$C_6$ alkoxy, $C_1$-$C_6$ haloalkyl, ($C_1$-$C_6$ alkoxy)carbonyl, nitro, cyano group or a halogen atom), examples of a $C_1$-$C_6$ alkyl group and and alkyl moiety of a $C_1$-$C_6$ haloalkyl group in R or P include methyl, ethyl, n-propyl, i-propyl, n-butyl, sec-butyl, tert-butyl and n-hexyl groups and the like; examples of a $C_1$-$C_6$ alkoxy group in R include methoxy, ethoxy, n-propoxy, i-propoxy, n-butoxy and hexyloxy groups and the like; examples of a halogen moiety of a $C_1$-$C_6$ haloalkyl group and a halogen atom in R or P include fluorine, chlorine, bromine and iodine atoms; examples of a ($C_1$-$C_6$ alkoxy)carbonyl group in R includes methoxycarbonyl, ethoxycarbonyl, n-butoxycarbonyl and 6-hexyloxycarbonyl groups and the like. Each of $R^1$ and $R^2$ represents a phenyl group optionally substituted with R, such as phenyl, 2-methylphenyl, 3-ethylphenyl, 4-hexylphenyl, 2,6-dimethylphenyl, 3-methoxyphenyl, 4-i-propoxyphenyl, 3-hexyloxyphenyl, 2-trifluoromethylphenyl, 3-difluoromethylphenyl, 2-methoxycarbonylphenyl, 2-ethoxycarbonylphenyl, 2-n-propoxycarbonylphenyl, 2-hexyloxycarbonylphenyl, 2-fluorophenyl, 2-chlorophenyl, 3-bromophenyl, 2,4-dichlorophenyl, 2,4,5-trichlorophenyl, 2-cyanophenyl and 3-nitrophenyl groups and the like.

Each of $R^1$ and $R^2$ represents a benzyl group optionally substituted with R, such as benzyl, 2-methylbenzyl, 3-ethylbenzyl, 4-hexylbenzyl, 2,6-dimethylbenzyl, 3-methoxybenzyl, 4-i-propoxybenzyl, 3-hexyloxybenzyl, 2-trifluoromethylbenzyl, 3-difluoromethylbenzyl, 2-methoxycarbonylbenzyl, 2-ethoxycarbonylbenzyl, 2-n-propoxycarbonylbenzyl, 2-hexyloxycarbonylbenzyl, 2-fluorobenzyl, 2-chlorobenzyl, 3-bromobenzyl, 2,4-dichlorobenzyl, 2,4,5-trichlorobenzyl, 2-cyanobenzyl and 3-nitrobenzyl groups and the like. Alternatively, $R^1$ and $R^2$ are bonded together at their ends to form an alkylene group optionally substituted with P, such as tetramethylene, pentamethylene, hexamethylene, 1,4-dimethyltetramethylene, 1,5-dimethylpentamethylene, 1-methylpentamethylene, 2-methylpentamethylene, 2-ethylpentamethylene, 2-butylpentamethylene, 2-hexyltetramethylene, 3-chloropentamethylene and 3-bromopentamethylene groups and the like. In addition, $R^1$ and $R^2$ are bonded together at their ends to form an alkylene group having one or more hetero atoms selected from a group consisting of oxygen, surfur and nitrogen, optionally substituted with P, such as the following groups:

$-CH_2-CH_2OCH_2CH_2-$ ,

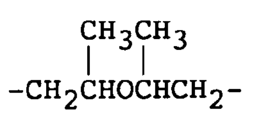

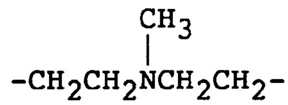 , 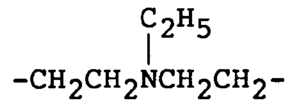

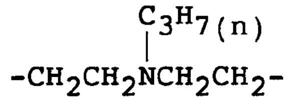 , 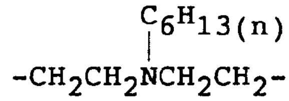

$-CH_2CH_2SCH_2CH_2-$ ,
$-CH_2CH_2SOCH_2CH_2-$
$-CH_2CH_2SO_2CH_2CH_2-$ ,

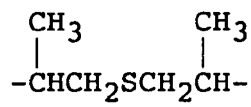

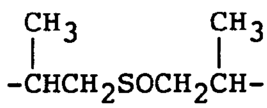 , 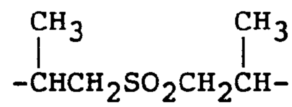

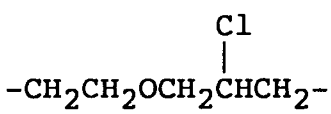 , 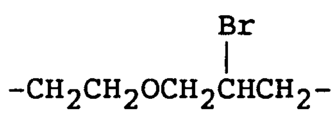

Moreover, $Y^4$ represents a phenyl group optionally substituted with R, such as phenyl, 2-methylphenyl, 3-ethylphenyl, 4-hexylphenyl, 2,6-dimethylphenyl, 3-methoxyphenyl, 4-i-propoxyphenyl, 3-hexyloxyphenyl, 2-trifluoromethylphenyl, 3-difluoromethylphenyl, 2-methoxycarbonylphenyl, 2-ethoxycarbonylphenyl, 2-n-propoxycarbonylphenyl, 2-hexyloxycarbonylphenyl, 2-fluorophenyl, 2-chlorophenyl, 3-bromophenyl, 2,4-dichlorophenyl, 2,4,5-trichlorophenyl, 2-cyanophenyl and, 3-nitrophenyl groups and the like; a phenoxy group optionally substituted with R, such as phenoxy, 2-methylphenoxy, 3-ethylphenoxy, 4-hexylphenoxy, 2,6-dimethylphenoxy, 3-methoxyphenoxy, 4-i-propoxyphenoxy, 3-hexyloxyphenoxy, 2-trifluoromethylphenoxy, 3-difluoromethylphenoxy, 2-methoxycarbonylphenoxy, 2-ethoxycarbonylphenoxy, 2-n-propoxycarbonylphenoxy, 2-hexyloxycarbonylphenoxy, 2-fluorophenoxy, 2-chlorophenoxy, 3-bromophenoxy, 2,4-dichlorophenoxy, 2,4,5-trichlorophenoxy, 2-cyanophenoxy and 3-nitrophenoxy groups and the like; a phenylthio group optionally substituted with R, such as phenylthio, 2-methylphenylthio, 3-ethylphenylthio, 4-hexylphenylthio, 2,6-dimethylphenylthio, 3-methoxyphenylthio, 4-i-propoxyphenylthio, 3-hexyloxyphenylthio, 2-trifluoromethylphenylthio, 3-difluoromethylphenylthio, 2-methoxycarbonylphenylthio, 2-ethoxycarbonylphenylthio, 2-n-propoxycarbonylphenylthio, 2-hexyloxycarbonylphenylthio, 2-fluorophenylthio, 2-chlorophenylthio, 3-bromophenylthio, 2,4-dichlorophenylthio, 2,4,5-trichlorophenylthio, 2-cyanophenylthio and 3-nitrophenylthio groups and the like; a benzyloxy group optionally substituted with R, such as benzyloxy, 2-methylbenzyloxy, 3-ethylbenzyloxy, 4-hexylbenzyloxy, 2,6-dimethylbenzyloxy, 3-methoxybenzyloxy, 4-i-propoxybenzyloxy, 3-hexyloxybenzyloxy, 2-trifluoromethylbenzyloxy, 3-difluoromethylbenzyloxy, 2-methoxycarbonylbenzyloxy, 2-ethoxycarbonylbenzyloxy, 2-n-propoxycarbonylbenzyloxy, 2-hexyloxycarbonylbenzyloxy, 2-fluorobenzyloxy, 2-chlorobenzyloxy, 3-bromobenzyloxy, 2,4-dichlorobenzyloxy, 2,4,5-trichlorobenzyloxy, 2-cyanobenzyloxy and 3-nitrobenzyloxy groups and the like; and a benzylthio group optionally substituted with R, such as benzylthio, 2-methylbenzylthio, 3-ethylbenzylthio, 4-hexylbenzylthio, 2,6-dimethylbenzylthio, 3-methoxybenzylthio, 4-i-propoxybenzylthio, 3-hexyloxybenzylthio, 2-trifluoromethylbenzylthio, 3-difluoromethylbenzylthio, 2-methoxycarbonylbenzylthio, 2-ethoxycarbonylbenzylthio, 2-n-propoxycarbonylbenzylthio, 2-hexyloxycarbonylbenzylthio, 2-fluorobenzylthio, 2-chlorobenzylthio, 3-bromobenzylthio, 2,4-dichlorobenzylthio, 2,4,5-trichlorophenylbenzylthio, 2-cyanobenzylthio and 3-nitrobenzylthio groups and the like.

A method for producing the present compound is as follows.

The present compound (I) can be produced by reacting a phenol or thiophenol derivative represented by the formula (II),

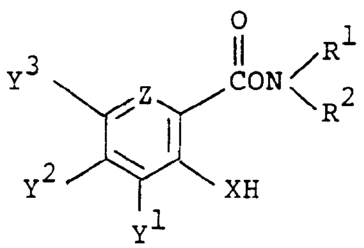

(II)

[wherein $R^1$, $R^2$, X, $Y^1$, $Y^2$, $Y^3$ and Z are as defined above], with a triazine derivative represented by the formula (III),

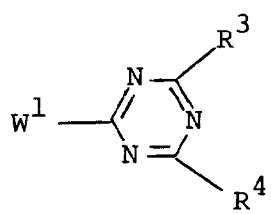

(III)

[wherein $R^3$ and $R^4$ are each as defined above and $W^1$ represents a halogen atom].

This reaction is usually carried out with or without a solvent in the presence of a base. The reaction temperature usually ranges from room temperature to the boiling point of the solvent, and the reaction time ranges from 10 minutes to 24 hours. Referring to the amounts of the reagents used for this reaction, the amount of the triazine derivative (III) is 1 to 3.0 equivalents based on 1 equivalent of the phenol derivative (II), and that of the base is 1 to 5 equivalents based on the same. The solvent includes aliphatic hydrocarbons (e.g. hexane, heptane, ligroin, petroleum ether), aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, diethylene glycol, dimethyl ether), ketones (e.g. acetone, methyl ethyl ketone, methyl isobutyl ketone, isophorone, cyclohexanone), esters (e.g. ethyl formate, ethyl acetate, butyl acetate), nitro compounds (e.g. nitroethane, nitrobenzene), nitriles (e.g. acetonitrile, isobutyronitrile), tertiary amines (e.g. pyridine, triethylamine, N,N-diethylaniline, tributylamine, N-methylmorpholine), acid amides (e.g. formamide, N,N-dimethylformamide, acetamide), sulfur compounds (e.g. dimethyl sulfoxide, sulfolane), liquid ammonia, water and mixtures thereof.

The base includes organic bases (e.g. pyridine, triethylamine, N,N-diethylaniline), inorganic bases (e.g. sodium hydroxide, potassium hydroxide, sodium carbonate, potassium carbonate, sodium hydride), alkali metal alkoxides (e.g. sodium methoxide, sodium ethoxide), etc.

After completion of the reaction, the reaction solution is after-treated as usual. That is, water is added to the solution which is then extracted with an organic solvent and concentrated, and if necessary, the product obtained is subjected to chromatography, distillation, recrystallization, etc. Thus, the desired present compound can be obtained.

The triazine derivative represented by the formula (III) can be produced according to J. Am. Chem. Soc., 73, 2981 (1951). The phenol derivative represented by the formula (II) can be produced according to EP 426476A1. Compound (I) includes its stereoisomers having a herbicidal activity.

Table 1 illustrates examples of the compound (I) which can be produced by the above procedures.

Table 1

Derivatives of the formula:

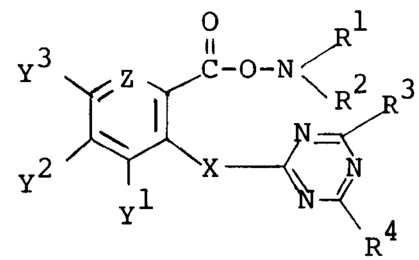

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (1) | H | H | H | H | H | O | CH | $OCH_3$ | $OCH_3$ | |
| (2) | H | H | H | H | H | O | N | $OCH_3$ | $OCH_3$ | |
| (3) | H | H | H | H | H | O | CCl | $OCH_3$ | $OCH_3$ | |
| (4) | H | H | H | H | H | O | CF | $OCH_3$ | $OCH_3$ | |
| (5) | H | $CH_3$ | H | H | H | O | CH | $OCH_3$ | $OCH_3$ | |
| (6) | H | $CH_3$ | H | H | H | O | N | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (7) | H | $CH_3$ | H | H | H | O | CCl | $OCH_3$ | $OCH_3$ | |
| (8) | H | $CH_3$ | H | H | H | O | CF | $OCH_3$ | $OCH_3$ | |
| (9) | $CH_3$ | $CH_3$ | H | H | H | O | CH | $OCH_3$ | $OCH_3$ | |
| (10) | $CH_3$ | $CH_3$ | H | H | H | S | CH | $OCH_3$ | $OCH_3$ | |
| (11) | $CH_3$ | $CH_3$ | H | H | H | O | CH | Cl | $OCH_3$ | |
| (12) | $CH_3$ | $CH_3$ | H | H | H | O | CH | $CH_3$ | $CH_3$ | |
| (13) | $C_2H_5$ | $C_2H_5$ | H | H | H | O | $CC_6H_5$ | $CH_3$ | $OCH_3$ | grassy oil |
| (14) | $CH_3$ | $CH_3$ | H | H | H | O | CH | $OC_2H_5$ | $OC_2H_5$ | |
| (15) | $C_2H_5$ | $C_2H_5$ | $CH_3$ | H | H | O | CH | $OCH_3$ | $OCH_3$ | $n_D^{25}$ 1.5112 |
| (16) | $CH_3$ | $CH_3$ | H | $CH_3$ | H | O | CH | $OCH_3$ | $OCH_3$ | |
| (17) | $CH_3$ | $CH_3$ | H | H | $CH_3$ | O | CH | $OCH_3$ | $OCH_3$ | |
| (18) | $CH_3$ | $CH_3$ | H | H | H | O | $CCH_3$ | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (19) | $CH_3$ | $CH_3$ | H | H | H | O | $CC_2H_5$ | $OCHF_2$ | $OCHF_2$ | |
| (20) | $CH_3$ | $CH_3$ | $OCH_3$ | H | H | O | CH | $OCH_3$ | $OCH_3$ | |
| (21) | $C_2H_5$ | $C_2H_5$ | H | $OCH_3$ | H | O | CH | $OCH_3$ | $OCH_3$ | $n_D^{25}$ 1.5025 |
| (22) | $CH_3$ | $CH_3$ | H | H | $OCH_3$ | O | CH | $OCH_3$ | $OCH_3$ | |
| (23) | $CH_3$ | $CH_3$ | H | H | H | O | $COCH_3$ | $OCH_3$ | $OCH_3$ | |
| (24) | $CH_3$ | $CH_3$ | F | H | H | O | CH | $OCH_3$ | $OCH_3$ | |
| (25) | $CH_3$ | $CH_3$ | H | F | H | O | CH | $OCH_3$ | $OCH_3$ | |
| (26) | $C_2H_5$ | $C_2H_5$ | H | H | F | O | CH | $OCH_3$ | $OCH_3$ | $n_D^{25}$ 1.5247 |
| (27) | $CH_3$ | $CH_3$ | H | H | H | O | CF | $OCH_3$ | $OCH_3$ | $n_D^{16}$ 1.5224 |
| (28) | $CH_3$ | $CH_3$ | H | H | F | O | CF | $OCH_3$ | $OCH_3$ | |
| (29) | $CH_3$ | $CH_3$ | Cl | H | H | O | CH | $OCH_3$ | $OCH_3$ | |
| (30) | $CH_3$ | $CH_3$ | H | Cl | H | O | CH | $OCH_3$ | $OCH_3$ | |
| (31) | $CH_3$ | $CH_3$ | H | H | Cl | O | CH | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (32) | $CH_3$ | $CH_3$ | H | H | H | O | CCl | $OCH_3$ | $OCH_3$ | $n_D^{16}$ 1.5373 |
| (33) | $CH_3$ | $CH_3$ | H | H | F | O | CCl | $OCH_3$ | $OCH_3$ | |
| (34) | $CH_3$ | $CH_3$ | H | H | H | S | CCl | $OCH_3$ | $OCH_3$ | |
| (35) | $CH_3$ | $CH_3$ | H | H | H | O | CBr | $OCH_3$ | $OCH_3$ | |
| (36) | $CH_3$ | $CH_3$ | H | H | H | O | CI | $OCH_3$ | $OCH_3$ | |
| (37) | $CH_3$ | $CH_3$ | H | H | H | O | COH | $OCH_3$ | $OCH_3$ | |
| (38) | $CH_3$ | $CH_3$ | H | H | H | O | CSH | $OCH_3$ | $OCH_3$ | |
| (39) | $CH_3$ | $CH_3$ | H | H | H | O | $CNO_2$ | $OCH_3$ | $OCH_3$ | |
| (40) | $CH_3$ | $CH_3$ | H | H | H | O | $CCH=CH_2$ | $OCH_3$ | $OCH_3$ | |
| (41) | $CH_3$ | $CH_3$ | H | H | H | O | $CC\equiv CH$ | $OCH_3$ | $OCH_3$ | |
| (42) | $CH_3$ | $CH_3$ | H | H | H | O | $COC_2H_5$ | $OCH_3$ | $OCH_3$ | |
| (43) | $CH_3$ | $CH_3$ | H | H | H | O | $COCH_2CH=CH_2$ | $OCH_3$ | $OCH_3$ | |
| (44) | $CH_3$ | $CH_3$ | H | H | H | O | $COCH_2C\equiv CH$ | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (45) | $CH_3$ | $CH_3$ | H | H | H | O | $CCF_3$ | $OCH_3$ | $OCH_3$ | |
| (46) | $CH_3$ | $CH_3$ | H | H | H | O | $CCH_2C(Cl)=CH_2$ | $OCH_3$ | $OCH_3$ | |
| (47) | $CH_3$ | $CH_3$ | H | H | H | O | $CC\equiv CI$ | $OCH_3$ | $OCH_3$ | |
| (48) | $CH_3$ | $CH_3$ | H | H | H | O | $COCF_3$ | $OCH_3$ | $OCH_3$ | |
| (49) | $CH_3$ | $CH_3$ | H | H | H | O | $CCH=CH_2$ | $OCH_3$ | $OCH_3$ | |
| (50) | $CH_3$ | $CH_3$ | H | H | H | O | $COC_2H_5$ | $OCH_3$ | $OCH_3$ | |
| (51) | $CH_3$ | $CH_3$ | H | H | H | O | $COCH_2CH=CHCI$ | $OCH_3$ | $OCH_3$ | |
| (52) | $CH_3$ | $CH_3$ | H | H | H | O | $COCH_2C\equiv Cl$ | $OCH_3$ | $OCH_3$ | |
| (53) | $CH_3$ | $CH_3$ | H | H | H | O | $CCH_2OCH_3$ | $OCH_3$ | $OCH_3$ | |
| (54) | $CH_3$ | $CH_3$ | H | H | H | O | CCN | $OCH_3$ | $OCH_3$ | |
| (55) | $CH_3$ | $CH_3$ | H | H | H | O | CCHO | $OCH_3$ | $OCH_3$ | |
| (56) | $CH_3$ | $CH_3$ | H | H | H | O | CCOOH | $OCH_3$ | $OCH_3$ | |
| (57) | $CH_3$ | $CH_3$ | H | H | H | O | $CCOOCH_3$ | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (58) | $CH_3$ | $CH_3$ | H | H | H | O | $CCOOCH_2C{\equiv}CH$ | $OCH_3$ | $OCH_3$ | |
| (59) | $CH_3$ | $CH_3$ | H | H | H | O | $CC_6H_5$ | $OCH_3$ | $OCH_3$ | $n_D^{23}$ 1.5598 |
| (60) | $CH_3$ | $CH_3$ | H | H | H | O | $COC_6H_5$ | $OCH_3$ | $OCH_3$ | |
| (61) | $CH_3$ | $CH_3$ | H | H | H | O | $CSC_6H_5$ | $OCH_3$ | $OCH_3$ | |
| (62) | $CH_3$ | $CH_3$ | H | H | H | O | $COCH_2C_6H_5$ | $OCH_3$ | $OCH_3$ | |
| (63) | $CH_3$ | $CH_3$ | H | H | H | O | $CSCH_2C_6H_5$ | $OCH_3$ | $OCH_3$ | |
| (64) | $CH_3$ | $CH_3$ | H | H | H | O | $CNH_2$ | $OCH_3$ | $OCH_3$ | |
| (65) | $CH_3$ | $CH_3$ | H | H | H | O | $CN(CH_3)_2$ | $OCH_3$ | $OCH_3$ | |
| (66) | $CH_3$ | $CH_3$ | H | H | H | O | $CN(CH_2CH{=}CH_2)_2$ | $OCH_3$ | $OCH_3$ | |
| (67) | $CH_3$ | $CH_3$ | H | H | H | O | $CN(CH_2C{\equiv}CH)_2$ | $OCH_3$ | $OCH_3$ | |
| (68) | $CH_3$ | $CH_3$ | H | H | H | O | $CCONH_2$ | $OCH_3$ | $OCH_3$ | |
| (69) | $CH_3$ | $CH_3$ | H | H | H | O | $CCON(C_2H_5)_2$ | $OCH_3$ | $OCH_3$ | |
| (70) | $CH_3$ | $CH_3$ | H | H | H | O | $CSCH_3$ | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (71) | $CH_3$ | $CH_3$ | H | H | H | O | $CSOC_2H_5$ | $OCH_3$ | $OCH_3$ | |
| (72) | $CH_3$ | $CH_3$ | H | H | H | O | $CSO_2C_3H_{7(n)}$ | $OCH_3$ | $OCH_3$ | |
| (73) | $CH_3$ | $CH_3$ | H | H | H | O | $CSCH_2CH{=}CH_2$ | $OCH_3$ | $OCH_3$ | |
| (74) | $CH_3$ | $CH_3$ | H | H | H | O | $CSCH_2C{\equiv}CH$ | $OCH_3$ | $OCH_3$ | |
| (75) | $CH_3$ | $CH_3$ | H | H | H | O | $CCH_2CH_2SCH_3$ | $OCH_3$ | $OCH_3$ | |
| (76) | $CH_3$ | $CH_3$ | H | H | H | O | $C(CH_2)_3SOCH_3$ | $OCH_3$ | $OCH_3$ | |
| (77) | $CH_3$ | $CH_3$ | H | H | H | O | $CCH_2SO_2CH_3$ | $OCH_3$ | $OCH_3$ | |
| (78) | $CH_3$ | $CH_3$ | H | H | H | S | $CC_6H_5$ | $OCH_3$ | $OCH_3$ | |
| (79) | $CH_3$ | $CH_3$ | H | H | H | O | 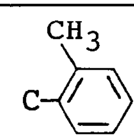 | $OCH_3$ | $OCH_3$ | |
| (80) | $CH_3$ | $CH_3$ | H | H | H | O | 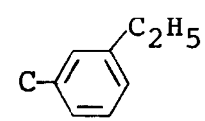 | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (81) | $CH_3$ | $CH_3$ | H | H | H | O | 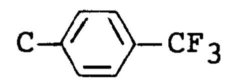 | $OCH_3$ | $OCH_3$ | |
| (82) | $CH_3$ | $CH_3$ | H | H | H | O | 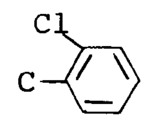 | $OCH_3$ | $OCH_3$ | |
| (83) | $CH_3$ | $CH_3$ | H | H | H | O | 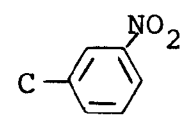 | $OCH_3$ | $OCH_3$ | |
| (84) | $CH_3$ | $CH_3$ | H | H | H | O | 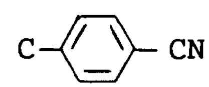 | $OCH_3$ | $OCH_3$ | |
| (85) | $CH_3$ | $CH_3$ | H | H | H | O | 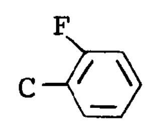 | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (86) | $CH_3$ | $CH_3$ | H | H | H | O | 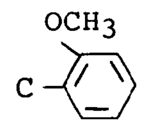 | $OCH_3$ | $OCH_3$ | |
| (87) | $CH_3$ | $CH_3$ | H | H | H | O | 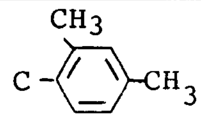 | $OCH_3$ | $OCH_3$ | |
| (88) | $CH_3$ | $CH_3$ | H | H | H | O | 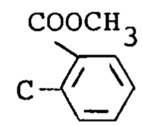 | $OCH_3$ | $OCH_3$ | |
| (89) | $CH_3$ | $CH_3$ | H | H | H | O | 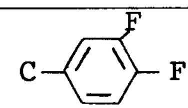 | $OCH_3$ | $OCH_3$ | |
| (90) | $CH_3$ | $CH_3$ | H | H | H | O | 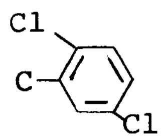 | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (91) | $CH_3$ | $CH_3$ | H | H | H | O | 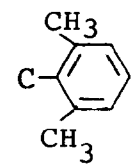 | $OCH_3$ | $OCH_3$ | |
| (92) | $CH_3$ | $CH_3$ | H | H | H | O | 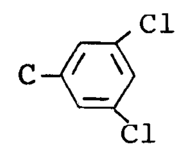 | $OCH_3$ | $OCH_3$ | |
| (93) | $CH_3$ | $CH_3$ | H | H | H | O | 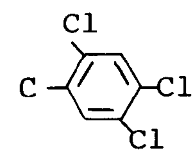 | $OCH_3$ | $OCH_3$ | |
| (94) | $CH_3$ | $CH_3$ | H | H | H | O | 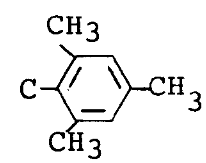 | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (95) | $CH_3$ | $C_2H_5$ | H | H | H | O | CF | $OCH_3$ | $OCH_3$ | |
| (96) | $CH_3$ | $C_2H_5$ | H | H | H | O | CCl | $OCH_3$ | $OCH_3$ | |
| (97) | $CH_3$ | $C_2H_5$ | H | H | H | O | $CC_6H_5$ | $OCH_3$ | $OCH_3$ | |
| (98) | $C_2H_5$ | $C_2H_5$ | H | H | H | O | CCl | $OCH_3$ | $OCH_3$ | |
| (99) | $C_2H_5$ | $C_2H_5$ | H | H | H | O | $CC_6H_5$ | $OCH_3$ | $OCH_3$ | $n_D^{23}$ 1.5416 |
| (100) | $C_3H_7$(i) | $C_3H_7$(i) | H | H | H | O | $CC_6H_5$ | $OCH_3$ | $OCH_3$ | |
| (101) | $C_3H_7$(n) | $C_3H_7$(n) | H | H | H | O | $CC_6H_5$ | $OCH_3$ | $OCH_3$ | m.p. 122.6°C |
| (102) | $C_4H_9$(n) | $C_4H_9$(n) | H | H | H | O | $CC_6H_5$ | $OCH_3$ | $OCH_3$ | |
| (103) | $C_5H_{11}$(n) | $C_5H_{11}$(n) | H | H | H | O | $CC_6H_5$ | $OCH_3$ | $OCH_3$ | |
| (104) | $C_6H_{13}$(n) | $C_6H_{13}$(n) | H | H | H | O | $CC_6H_5$ | $OCH_3$ | $OCH_3$ | |
| (105) | $C_6H_{13}$(n) | $C_6H_{13}$(n) | H | H | H | O | CCl | $OCH_3$ | $OCH_3$ | |
| (106) | $C_6H_{13}$(n) | $C_6H_{13}$(n) | H | H | H | O | CF | $OCH_3$ | $OCH_3$ | |
| (107) | H | $C_3H_7$(i) | H | H | H | O | CCl | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (108) | H | $C_3H_7$(i) | H | H | H | O | $CC_6H_5$ | $OCH_3$ | $OCH_3$ | |
| (109) | H | $C_4H_9$(t) | H | H | H | O | CCl | $OCH_3$ | $OCH_3$ | |
| (110) | H | $C_4H_9$(t) | H | H | H | O | $CC_6H_5$ | $OCH_3$ | $OCH_3$ | |
| (111) | $CH_2CH{=}CH_2$ | $CH_2CH{=}CH_2$ | H | H | H | O | CCl | $OCH_3$ | $OCH_3$ | |
| (112) | $CH_2CH{=}CH_2$ | $CH_2CH{=}CH_2$ | H | H | H | O | $CC_6H_5$ | $OCH_3$ | $OCH_3$ | |
| (113) | $CH_2C{\equiv}CH$ | $CH_2C{\equiv}CH$ | H | H | H | O | CF | $OCH_3$ | $OCH_3$ | |
| (114) | $CH_2C{\equiv}CH$ | $CH_2C{\equiv}CH$ | H | H | H | O | $CC_6H_5$ | $OCH_3$ | $OCH_3$ | |
| (115) | $CH_2CH_2Cl$ | $CH_2CH_2Cl$ | H | H | H | O | $CC_6H_5$ | $OCH_3$ | $OCH_3$ | |
| (116) | $CH_2CH_2OCH_3$ | $CH_2CH_2OCH_3$ | H | H | H | O | $CC_6H_5$ | $OCH_3$ | $OCH_3$ | |
| (117) | $CH_2CH_2O$ 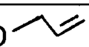 | $CH_2CH_2O$ 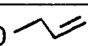 | H | H | H | O | $CC_6H_5$ | $OCH_3$ | $OCH_3$ | |
| (118) | $CH_2CH_2O$ 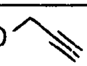 | $CH_2CH_2O$ 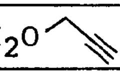 | H | H | H | O | $CC_6H_5$ | $OCH_3$ | $OCH_3$ | |
| (119) | $CH_2COOCH_3$ | $CH_2COOCH_3$ | H | H | H | O | $CCH_3$ | $OCH_3$ | $OCH_3$ | |
| (120) | $CH_3$ | $CH_2COOCH_3$ | H | H | H | O | $CC_3H_7$(n) | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (121) | $CH_3$ | $CH_3$<br>$CHCOOCH_3$ | H | H | H | O | CCl | $OCH_3$ | $OCH_3$ | |
| (122) | $CH_2COO$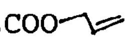 | $CH_2COO$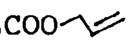 | H | H | H | O | $CCF_3$ | $OCH_3$ | $OCH_3$ | |
| (123) | $CH_2COO$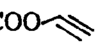 | $CH_2COO$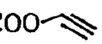 | H | H | H | O | CCl | $OCH_3$ | $OCH_3$ | |
| (124) | $CH_2CN$ | $CH_2CN$ | H | H | H | O | $COCH_3$ | $OCH_3$ | $OCH_3$ | |
| (125) | $CH_2CH_2CN$ | $CH_2CH_2CN$ | H | H | H | O | CCl | $OCH_3$ | $OCH_3$ | |
| (126) | $CH_2CONH_2$ | $CH_2CONH_2$ | H | H | H | O | N | $OCH_3$ | $OCH_3$ | |
| (127) | $CH_2CON(CH_3)_2$ | $CH_2CON(CH_3)_2$ | H | H | H | O | $CC_6H_5$ | $OCH_3$ | $OCH_3$ | |
| (128) | $CH_2CH_2SCH_3$ | $CH_2CH_2SCH_3$ | H | H | H | O | $CC_6H_5$ | $OCH_3$ | $OCH_3$ | |
| (129) | $CH_2CH_2SOCH_3$ | $CH_2CH_2SOCH_3$ | H | H | H | O | CCl | $OCH_3$ | $OCH_3$ | |
| (130) | $CH_2CH_2SO_2CH_3$ | $CH_2CH_2SO_2CH_3$ | H | H | H | O | CF | $OCH_3$ | $OCH_3$ | |
| (131) | H | cyclohexyl | H | H | H | O | CCl | $CH_3$ | $OCH_3$ | |
| (132) | H | cyclohexyl | H | H | H | O | $CC_6H_5$ | $OCH_3$ | $OCH_3$ | |
| (133) | H | cyclohexyl | H | H | H | O | CF | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (134) | H | 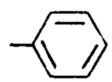 | H | H | H | O | $CC_6H_5$ | $OCH_3$ | $OCH_3$ | |
| (135) | H | 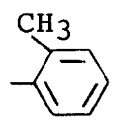 | H | H | H | O | $CC_6H_5$ | $OCH_3$ | $OCH_3$ | |
| (136) | H | 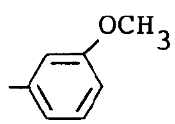 | H | H | H | O | $CC_6H_5$ | $OCH_3$ | $OCH_3$ | |
| (137) | $CH_3$ | 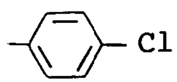 | H | H | H | O | CCl | $OCH_3$ | $OCH_3$ | |
| (138) | $CH_3$ | 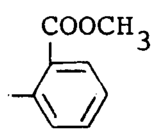 | H | H | H | O | CF | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (139) | $CH_3$ | 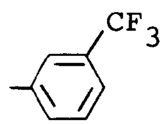 | H | H | H | O | CCl | $OCH_3$ | $OCH_3$ | |
| (140) | 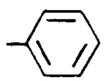 | 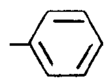 | H | H | H | O | $CC_6H_5$ | $OCH_3$ | $OCH_3$ | |
| (141) | 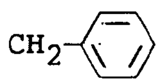 | 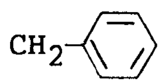 | H | H | H | O | CCl | $OCH_3$ | $OCH_3$ | |
| (142) | 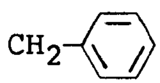 | 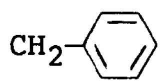 | H | H | H | O | $CC_6H_5$ | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (143) | $(CH_2)_4$ | | H | H | H | O | $CC_6H_5$ | $OCH_3$ | $OCH_3$ | $n_D^{24}$ 1.5498 |
| (144) | $(CH_2)_4$ | | H | H | H | O | CCl | $OCH_3$ | $OCH_3$ | |
| (145) | $(CH_2)_4$ | | H | H | H | S | CF | $OCH_3$ | $OCH_3$ | |
| (146) | $(CH_2)_5$ | | H | H | H | O | $CC_6H_5$ | $OCH_3$ | $OCH_3$ | |
| (147) | $(CH_2)_5$ | | H | H | H | S | CCl | $OCH_3$ | $OCH_3$ | |
| (148) | $(CH_2)_6$ | | H | H | H | O | $CC_6H_5$ | $OCH_3$ | $OCH_3$ | |
| (149) | $CH_3$ $CH_3$<br>$-CHCH_2CH_2CH-$ | | H | H | H | O | CCl | $OCH_3$ | $OCH_3$ | |
| (150) | $CH_3$ $CH_3$<br>$-CHCH_2CH_2CH-$ | | H | H | H | O | $CC_6H_5$ | $OCH_3$ | $OCH_3$ | |
| (151) | $CH_3$ $CH_3$<br>$-CHCH_2CH_2CH_2CH-$ | | H | H | H | O | $CC_6H_5$ | $OCH_3$ | $OCH_3$ | |

| Compound No. | $R^1$ | $R^2$ | $Y^1$ | $Y^2$ | $Y^3$ | X | Z | $R^3$ | $R^4$ | Physical properties (melting point, refractive index) |
|---|---|---|---|---|---|---|---|---|---|---|
| (152) | $-CH(CH_3)CH_2CH_2CH_2CH(CH_3)-$ | | H | H | H | O | CCl | $OCH_3$ | $OCH_3$ | |
| (153) | $-CH_2CH(CH_3)CH_2CH_2CH_2-$ | | H | H | H | O | CF | $OCH_3$ | $OCH_3$ | |
| (154) | $-CH(CH_3)CH_2CH_2CH_2CH_2-$ | | H | H | H | O | $CC_6H_5$ | $OCH_3$ | $OCH_3$ | |
| (155) | $-CH(C_2H_5)CH_2CH_2CH(CH_3)-$ | | H | H | H | O | CCl | $OCH_3$ | $OCH_3$ | |
| (156) | $-CH(C_2H_5)CH_2CH_2CH_2CH(C_2H_5)-$ | | H | H | H | O | $CC_6H_5$ | $OCH_3$ | $OCH_3$ | |
| (157) | $-CH_2CH_2CH(Cl)CH_2CH_2-$ | | H | H | H | O | CCl | $OCH_3$ | $OCH_3$ | |
| (158) | $-CH_2CH_2SO_2CH_2CH_2-$ | | H | H | H | O | $CC_6H_5$ | $OCH_3$ | $OCH_3$ | |

The present compound (I) has an excellent herbicidal activity and some of them have excellent selectivity between crops and weeds, when used in herbicidally effective amounts.

That is, the present compound, when used for foliar treatment and soil treatment in upland fields, exhibits a herbicidal activity against various weeds in question. Also, the present compound (I), when used for flooding treatment in paddy fields, exhibits a herbicidal activity against various weeds in question.

The present compound (I) can control a wide range of weeds generated in crop lands or non-crop lands, can be applied in low dosage rates, has a broad herbicidal spectrum and also can safely be used for no-till cultivation in soybean fields, peanut fields, corn fields, etc.

As weeds which can be controlled by the present compound, there are mentioned for example broad-leaved weeds such as wild buckwheat (Polygonum convolvulus), pale smartweed (Polygonum lapathifolium), common purslane (Portulaca oleracea), common chickweed (Stellaria media), common lambsquarters (Chenopodium album), redroot pigweed (Amaranthus retroflexus), radish (Raphanus sativus), wild mustard (Sinapis arvensis), shepherd's-purse (Capsella bursa-pastoris), hemp sesbania (sesbania exaltata), sicklepod (Cassia obtusifolia), velvetleaf (Abutilon theophrasti), prickly sida (Sida spinosa), field pansy (Viola arvensia), cleavers (Galium aparine), ivyleaf morningglory (Ipomoea hederacea), tall morningglory (Ipomoea purpurea), field bindweed (Convolvulus arvensis), red deadnettle (Lamium purpureum), henbit (Lamium amplexicaure), jimsonweed (Datura stramonium), black nightshade (Solanum nigrum), birdseye speedwell (Veronica persica), cocklebur (Xanthium strumarium), sunflower (Helianthus annuus), scentless chamomile (Matricaria perforata), corn marigold (Chrysanthemum seqetum), etc.; Gramineae weeds such as Japanese millet (Echinochloa frumentacea), barnyardgrass (Echinochloa crus-qalli), green foxtail (Setaria viridis), giant foxtail (Setaria faberi), large crabgrass (Digitaria sanquinalis), annual bluegrass (Poa annua), blackgrass (Alopecurus myosuroides), oat (Avena sativa), wild oat (Avena fatua), johnsongrass (Sorghum halepense), quackgrass (Agropyron repens), downy brome (Bromus tectorum), bermudagrass (Cynodon dactylon), etc.; Commelinaceae weeds such as dayflower (Commelina communis), etc.; and Cyperaceae weeds such as rice flatsedge (Cyperus iria), purple nutsedge (Cyperus rotundus), yellow nutsedge (Cyperus esculentus), etc. In addition, some of the present compounds give such no phytotoxicity as becoming a problem to main crops such as corn, wheat, barley, rice, soybean, cotton, beet, etc.

In flooding treatment in paddy fields, the present compounds exhibit a herbicidal activity against gramineous weeds such as barnyardgrass (Echinochloa oryzicola), etc.; broad-leaved weeds such as false pimpernel (Lindernia procumbens), indian toothcup (Rotala indica), waterwort (Elatine triandra), Ammannia multiflora, etc.; Cyperaceae weeds such as smallflower umbrellaplant (Cyperus difformis), bulrush (Scirpus juncoides), slender spikerush (Eleocharis acicularis), water nutgrass (Cyperus serotinus), etc.; monochoria (Monochoria vaginalis), arrowhead (Sagittaria pygmaea), etc.

When the present compound (I) is used as an active ingredient for herbicides, it is usually formulated before use into emulsifiable concentrates, wettable powders, suspension formulations, granules, water-dispersible granules, etc. by mixing with solid carriers, liquid carriers, surface active agents or other auxiliaries for formulation.

The content of the compound (I) as an active ingredient in these preparations is normally within a range of about 0.001 to 90% by weight, preferably of about 0.003 to 80% by weight.

Examples of the solid carriers are fine powders or granules of kaolin clay, attapulgite clay, bentonite, terra alba, pyrophyllite, talc, diatomaceous earth, calcite, walnut shell powders, urea, ammonium sulfate and synthetic hydrated silicon dioxide, etc.

Examples of the liquid carriers are aromatic hydrocarbons (e.g. xylene, methylnaphthalene), alcohols (e.g. isopropanol, ethylene glycol, cellosolve), ketones (e.g. acetone, cyclohexanone, isophorone), vegetable oils (soybean oil, cotton seed oil), dimethyl sulfoxide, N,N-dimethylformamide, acetonitrile, water, etc.

Examples of the surface active agents used for emulsification, dispersion or spreading, etc. are anionic surface active agents such as salts of alkyl sulfates, alkylsulfonates, alkylarylsulfonates, dialkyl sulfosuccinates, salts of polyoxyethylene alkylaryl ether phosphoric acid esters, etc., and nonionic surface active agents such as polyoxyethylene alkyl ethers, polyoxyethylene alkylaryl ethers, polyoxyethylene polyoxypropylene block copolymers, sorbitan fatty acid esters, polyoxyethylene sorbitan fatty acid esters, etc.

Examples of other auxiliaries for formulation are lignosulfonates, alginates, polyvinyl alcohol, gum arabic, CMC (carboxymethyl cellulose), PAP (isopropyl acid phosphate), etc.

The present compound (I) is usually formulated and used in soil treatment, foliar treatment or flooding treatment before or after emergence of weeds. The soil treatment includes soil surface treatment, soil incorporation treatment, etc. The foliar treatment includes, in addition to treatment of plants from above, directed treatment in which treatment is limited to weeds only so as not to adhere to crops.

Build-up of the herbicidal activity of the present compound (I) can be expected by using them in mixture with other herbicides. Further, the present compound (I) can also be used in mixture with insecticides, acaricides, nematocides, fungicides, plant growth regulators, fertilizers, soil improvers, etc.

The present compound (I) can be used as an active ingredient for herbicides used in paddy fields, ridges of paddy fields, plowed fields, non-plowed fields, orchards, pastures, turfs, forests and non-agricultural fields, etc.

When the present compound (I) is used as an active ingredient for herbicides, their dosage rate varies with weather conditions, preparation forms, when, how and where the treatment is carried out, weeds species to be controlled, crops species to be protected, etc. Usually, however, the dosage rate is from 0.003 gram to 100 grams of the active ingredient per are, preferably from 0.01 gram to 80 grams of the active ingredient per are.

The herbicidal composition of the invention formulated in the form of an emulsifiable concentrate, a wettable powder, a suspension formulation or water dispersible granules may ordinarily be employed by diluting it with water at a volume of about 1 to 10 liters per are (if necessary, auxiliaries such as a spreading agent are added to the water). The granules are usually applied as they are without being diluted.

The spreading agent includes, in addition to the foregoing surface active agents, substances such as polyoxyethylene resin acids (esters), lignosulfonates, abietates, dinaphthylmethanedisulfonates, paraffin, etc.

Production example 1

0.4 Gram of 60% sodium hydride in oil was suspended in 10 ml of N,N-dimethylformamide and cooled at 0°C - 5°C. A solution of 2.05 g of 3-chloro-2-(N,N-dimethylaminooxycarbonyl)phenol in 5 ml of N,N-dimethylformamide was added. After stirring at 0°C - 5°C for 30 minutes, a solution of 1.67 g of 4,6-dimethoxy-2-chloro-1,3,5-triazine in 5 ml of N,N-dimethylformamide was added. The reaction solution was raised to room temperature and kept at the same temperature for 2 hours with stirring. The reaction solution was poured into saturated aqueous sodium chloride and extracted with ethyl acetate. The organic layer was washed with saturated aqueous sodium chloride and dried over anhydrous magnesium sulfate. The solvent was removed under reduced pressure, and the residue obtained was subjected to thin-layer chromatography on silica gel (mfd. by Merck Art 5717) with chloroform/ethyl acetate (5:1 v/v) as eluents to obtain 0.7 g of 2,4-dimethoxy-6-{2-(N,N-dimethylaminooxycarbonyl)-3-chlorophenoxy}- 1,3,5-triazine [present compound (32)].

$^1$H-NMR ($CDCl_3$) δ: 2.77 (s, 6H), 3.95 (s, 6H), 7.01 - 7.40 (m, 3H)

$n_D^{16}$: 1.5373

Production example 2

0.2 Gram of 60% sodium hydride in oil was suspended in 10 ml of N,N-dimethylformamide and cooled at 0°C - 5°C. A solution of 1.24 g of 3-phenyl-2-(N,N-dimethylaminooxycarbonyl)phenol in 3 ml of N,N-dimethylformamide was added. After stirring at 0°C - 5°C for 30 minutes, a solution of 0.85 g of 4,6-dimethoxy-2-chloro-1,3,5-triazine in 2 ml of N,N-dimethylformamide was added. The reaction solution was raised to room temperature and kept at the same temperature for 3 hours with stirring. The reaction solution was poured into saturated aqueous sodium chloride and extracted with ethyl acetate. The organic layer was washed with saturated aqueous sodium chloride and dried over anhydrous magnesium sulfate. The solvent was removed under reduced pressure, and the residue obtained was subjected to thin-layer chromatography on silica gel (mfd. by Merck Art 5717) with chloroform/ethyl acetate (5:1 v/v) as eluents to obtain 0.5 g of 2,4-dimethoxy-6-{2-(N,N-dimethylaminooxycarbonyl)-3-phenylphenoxy}- 1,3,5-triazine [present compound (59)].

$n_D^{23}$: 1.5598

$^1$H-NMR ($CDCl_3$) δ: 2.38 (s, 6H), 3.94 (s, 6H), 7.07 - 7.49 (m, 8H)

Production example 3

According to the procedure shown in Production Example 1, 2,4-dimethoxy-6-{2-(N,N-dimethylaminooxycarbonyl)-3-phenylphenylthio}-1,3,5-triazine can be obtained by reacting 3-phenyl-2-(N,N-dimethylaminooxycarbonyl)thiophenol with 4,6-dimethoxy-2-chloro-1,3,5-triazine.

Formulation examples are shown below. In the examples, the present compound (I) is shown by Compound No. in Table 1, and parts are by weight.

Formulation example 1

Ten parts of the present compound (13), (15), (21), (26), (27), (32), (59), (99) or (101), 14 parts of polyoxyethylene styrylphenyl ether, 6 parts of calcium dodecylbenzenesulfonate, 40 parts of xylene and 30 parts of cyclohexanone are well mixed to obtain an emulsifiable concentrate.

Formulation example 2

Twenty five parts of the present compound (13), (15), (21), (26), (27), (32), (59), (99) or (101), 3 parts of polyoxyethylene sorbitan monooleate, 3 parts of CMC and 69 parts of water are mixed and wet-pulverized until the particle size decreases to 5 microns or less. Thus, a suspension formulation is obtained.

Formulation example 3

Fifty parts of the present compound (13), (15), (21), (26), (27), (32), (59), (99) or (101), 3 parts of calcium lignosulfonate, 2 parts of sodium lauryl sulfate and 45 parts of synthetic hydrated silicon dioxide are well mixed while being powdered to obtain a wettable powder.

Formulation example 4

Two parts of the present compound (13), (15), (21), (26), (27), (32), (59), (99) or (101), 1 part of synthetic hydrated silicon dioxide, 2 parts of calcium lignosulfonate, 30 parts of bentonite and 65 parts of kaolin clay are well pulverized and mixed. The resulting mixture is well kneaded with water, granulated and dried to obtain a granule.

That the present compounds are useful as an active ingredient for herbicides is shown by test examples. In the examples, the present compound (I) is shown by Compound No. in Table 1 and compounds used for comparison are shown by Compound symbol in Table 2.

Table 2

| Compound symbol | Structural formula | Remarks |
|---|---|---|
| (A) | 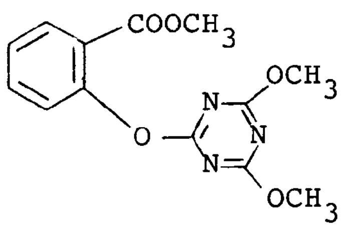 | Japanese Patent Application Kokai No. 63-258467 |
| (B) | 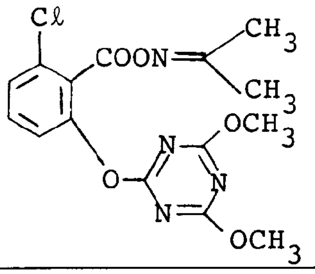 | EP 346789 |
| (C) | 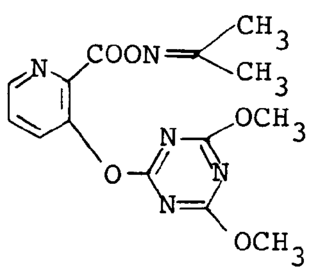 | EP 372329 |

The herbicidal activity and phytotoxicity were evaluated in six stages, 5, 4, 3, 2, 1, 0, by comparing germination and growth of test plants with those untreated.

[0]: the states of germination and growth of test plants showed no difference.

[5]: test plants either completely died or germination/growth were totally inhibited.

Test example 1 Foliar treatment test in upland field soil

Cylindrical plastic pots of 10 cm in diameter and 10 cm in depth were filled with upland field soil, and the seeds of tall morningglory and radish were sowed in the respective pots and cultivated for 8 days in a greenhouse.

Thereafter, the test compounds were formulated into emulsifiable concentrates according to Formulation example 1 and the prescribed amount of each emulsifiable concentrate was diluted with a spreading agent-containing water corresponding to 10 liters/are and uniformly applied from above onto the whole foliar portion of the test plants by means of an automatic sprayer. After application, the test plants were cultivated for 19 days in a greenhouse, and the herbicidal activity was examined.

The results are shown in Table 3.

Table 3

| Test compound | Dosage rate of active ingredient (gram/are) | Herbicidal activity | |
|---|---|---|---|
| | | Tall morning glory | Radish |
| (32) | 1.25 | 4 | 5 |
| (59) | 1.25 | 4 | 5 |
| (27) | 1.25 | 5 | 5 |
| (A) | 1.25 | 1 | 1 |
| (C) | 1.25 | 2 | 1 |

Test example 2 Soil treatment test in upland field soil

Vats of 33 x 23 $cm^2$ in area and 11 cm in depth were filled with upland field soil, and the seeds of wheat, barley, birdseye speedwell, common chickweed, wild oat and blackgrass were sowed in the respective vats and covered with soil in a thickness of 1 to 2 cm. The test compounds were formulated into emulsifiable concentrates according to Formulation example 1, and the prescribed amount of each emulsifiable concentrate was diluted with water corresponding to 10 liters/are and uniformly applied onto the whole soil surface by means of an automatic sprayer. After application, the test plants were cultivated for 25 days in a greenhouse, and the herbicidal activity and phytotoxicity were examined. The results are shown in Table 4.

Table 4

| Test compound | Dosage rate of active ingredient (gram/are) | Phototoxity | | Herbicidal activity | | | |
|---|---|---|---|---|---|---|---|
| | | Wheat | Barley | Birdseye speedwell | Common chickweed | Wild oat | Black-grass |
| (59) | 0.32 | 1 | 0 | 5 | 5 | 4 | 4 |
| (A) | 1.25 | 0 | 2 | 3 | 0 | 1 | 2 |

Test example 3 Soil treatment test in upland field soil

Vats of 33 x 23 $cm^2$ in area and 11 cm in depth were filled with upland field soil, and the seeds of beat, downy brome, annual bluegrass, birdseye speedwell were sowed in the respective vats and covered with soil in a thickness of 1 to 2 cm. The test compounds were formulated into emulsifiable concentrates according to Formulation example 1, and the prescribed amount of each emulsifiable concentrate was diluted with water

corresponding to 10 liters/are and uniformly applied onto the whole soil surface by means of an automatic sprayer.

After application, the test plants were cultivated for 25 days in a greenhouse, and the herbicidal activity and phytotoxicity were examined. The results are shown in Table 5.

Table 5

| Test compound | Dosage rate of active ingredient (gram/are) | Phytotoxicity | Herbicidal activity | | |
|---|---|---|---|---|---|
| | | Beat | Birdseye speedwell | Downy brome | Annual bluegrass |
| (27) | 1.25 | 0 | 4 | 4 | 4 |
| (32) | 1.25 | 0 | 4 | 4 | 4 |
| (A) | 1.25 | 2 | 3 | 3 | 3 |

Test example 4 Soil surface treatment test in upland field soil

Cylindrical plastic pots of 10 cm in diameter and 10 cm in depth were filled with upland field soil, and the seeds of Japanese millet was sowed in the respective pots and covered with soil. The test compounds were formulated into emulsifiable concentrates according to Formulation example 1, and the prescribed amount of each emulsifiable concentrate was diluted with water corresponding to 10 liters/are and uniformly applied onto

the whole soil surface by means of an automatic sprayer. After application, the test plants were cultivated for 19 days in a greenhouse, and the herbicidal activity was examined. The results are shown in Table 6.

Table 6

| Test compound | Dosage rate of active ingredient (gram/are) | Herbicidal activity |
|---|---|---|
| | | Japanese millet |
| (21) | 1.25 | 4 |
| (59) | 1.25 | 5 |
| (27) | 1.25 | 4 |
| (B) | 1.25 | 2 |
| (C) | 1.25 | 0 |

## Claims

1. A triazine derivative represented by the formula,

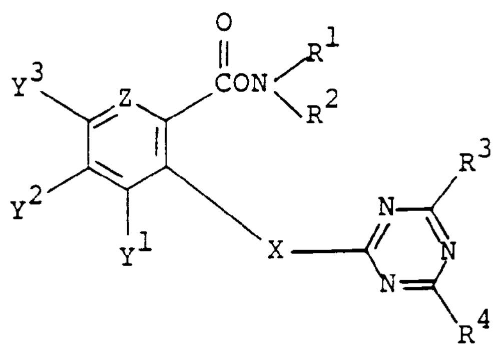

(I)

wherein each of $R^1$ and $R^2$, which may be the same or different, represents a hydrogen atom; a $C_1$-$C_6$ alkyl group; a $C_3$-$C_6$ alkenyl group; a $C_3$-$C_6$ alkynyl group; a halo-$C_1$-$C_6$ alkyl group; a $C_1$-$C_6$ alkoxy-$C_1$-$C_6$ alkyl group; a $C_3$-$C_6$ alkenyloxy-$C_1$-$C_6$ alkyl group; a $C_3$-$C_6$ alkynyloxy-$C_1$-$C_6$ alkyl group; a $C_1$-$C_6$ alkoxy-carbonyl-$C_1$-$C_6$ alkyl group; a $C_3$-$C_6$ alkenyloxycarbonyl-$C_1$-$C_6$ alkyl group; a $C_3$-$C_6$ alkynyloxycarbonyl-$C_1$-$C_6$ alkyl group; a cyano-$C_1$-$C_6$ alkyl group; a cyclo-$C_3$-$C_8$ alkyl group; a group represented by the formula,

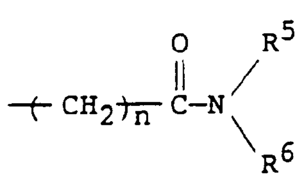

(in which each of $R^5$ and $R^6$, which may be the same or different, represents a hydrogen atom, a $C_1$-$C_6$

alkyl group, a $C_3$-$C_6$ alkenyl group or a $C_3$-$C_6$ alkynyl group, and n represents an integer of 1, 2, 3 or 4); a group represented by the formula,

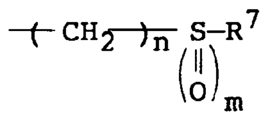

(in which n is as defined above, $R^7$ represents a $C_1$-$C_6$ alkyl group, a $C_3$-$C_6$ alkenyl group or a $C_3$-$C_6$ alkynyl group, and m represents an integer of 0, 1 or 2); a phenyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; or a benzyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms, or $R^1$ and $R^2$ are bonded together at their ends to form a $C_4$-$C_7$ alkylene group optionally substituted with at least one member selected from the group consisting of $C_1$-$C_6$ alkyl groups and halogen atoms or $R^1$ and $R^2$ are bonded together at their ends to form a $C_3$-$C_6$ alkylene group containing a hetero atom optionally substituted with at least one member selected from the group consisting of $C_1$-$C_6$ alkyl groups and halogen atoms (the hetero atom in $R^1$ and $R^2$ means a nitrogen atom, an oxygen atom or a sulfur atom),

each of $R^3$ and $R^4$, which may be the same or different, represents a $C_1$-$C_6$ alkyl group; a $C_1$-$C_6$ alkoxy group; a halo-$C_1$-$C_6$ alkoxy group; or a halogen atom,

X represents an oxygen atom or a sulfur atom,

Z represents CH, N or $CY^4$,

each of $Y^1$, $Y^2$ and $Y^3$, which may be the same or different, represents a hydrogen atom; a halogen atom; a $C_1$-$C_6$ alkyl group; a $C_1$-$C_6$ alkoxy group; a nitro group; a cyano group; or a phenyl group, and

$Y^4$ represents a hydroxy group; a mercapto group; a nitro group; a halogen atom; a $C_1$-$C_6$ alkyl group; a $C_2$-$C_6$ alkenyl group; a $C_2$-$C_6$ alkynyl group; a $C_1$-$C_6$ alkoxy group; a $C_3$-$C_6$ alkenyloxy group; a $C_3$-$C_6$ alkynyloxy group; a halo-$C_1$-$C_6$ alkyl group; a halo-$C_2$-$C_6$ alkenyl group; a halo-$C_2$-$C_6$ alkynyl group; a halo-$C_1$-$C_6$ alkoxy group; a halo-$C_3$-$C_6$ alkenyloxy group; a halo-$C_3$-$C_6$ alkynyloxy group; a $C_1$-$C_6$ alkoxy-$C_1$-$C_6$ alkyl group; a $C_3$-$C_6$ alkenyloxy-$C_1$-$C_6$ alkyl group; a $C_3$-$C_6$ alkynyloxy-$C_1$-$C_6$ alkyl group; a cyano group; a formyl group; a carboxyl group; a ($C_1$-$C_6$ alkoxy)carbonyl group; a ($C_3$-$C_6$ alkenyloxy)carbonyl group; a ($C_3$-$C_6$ alkynyloxy)carbonyl group; a phenyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a phenoxy group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a phenylthio group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro, cyano groups and halogen atoms; a benzyloxy group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a benzylthio group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms, a group represented by the formula,

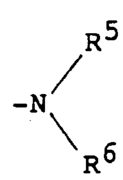

(in which each of $R^5$ and $R^6$ as described above); a group represented by the formula,

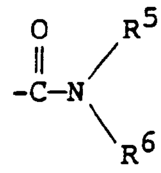

(in which $R^5$ and $R^6$ are as defined above); a group represented by the formula,

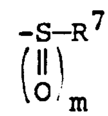

(in which $R^7$ and m are as described above); a group represented by the formula

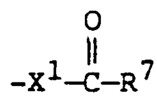

(in which $R^7$ is as defined above and $X^1$ is an oxygen atom or a sulfuratom);or a group represented by the formula,

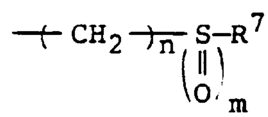

(in which $R^7$, m and n are as defined above).

2. A triazine derivative represented by the formula,

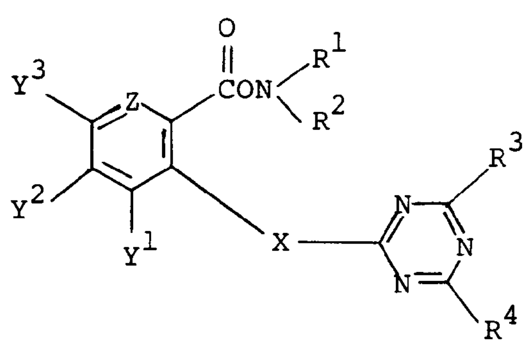

(I)

wherein each of $R^1$ and $R^2$, which may be the same or different, represents a hydrogen atom; a $C_1$-$C_6$ alkyl group; a $C_3$-$C_6$ alkenyl group; a $C_3$-$C_6$ alkynyl group; a halo-$C_1$-$C_6$ alkyl group; a $C_1$-$C_6$ alkoxy-$C_1$-$C_6$ alkyl group; a $C_3$-$C_6$ alkenyloxy-$C_1$-$C_6$ alkyl group; a $C_3$-$C_6$ alkynyloxy-$C_1$-$C_6$ alkyl group; a $C_1$-$C_6$ alkoxy-carbonyl-$C_1$-$C_6$ alkyl group; a $C_3$-$C_6$ alkenyloxycarbonyl-$C_1$-$C_6$ alkyl group; a $C_3$-$C_6$ alkynyloxycarbonyl-$C_1$-$C_6$ alkyl group; a cyano-$C_1$-$C_6$ alkyl group; a cyclo-$C_3$-$C_8$ alkyl group; a phenyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; or a benzyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms, or $R^1$ and $R^2$ are bonded together at their ends to form a $C_4$-$C_7$ alkylene group optionally substituted with at least one member selected from the group consisting of $C_1$-$C_6$ alkyl groups and halogen atoms, or $R^1$ and $R^2$ are bonded together at their ends to form a $C_3$-$C_6$ alkylene group containing a hetero atom

optionally substituted with at least one member selected from the group consisting of $C_1$-$C_6$ alkyl groups and halogen atoms (the hetero atom in $R^1$ and $R^2$ means a nitrogen atom, an oxygen atom or a sulfur atom),

each of $R^3$ and $R^4$, which may be the same or different, represents a $C_1$-$C_6$ alkyl group; a $C_1$-$C_6$ alkoxy group; a halo-$C_1$-$C_6$ alkoxy group; or a halogen atom,

X represents an oxygen atom or a sulfur atom,

Z represents CH, N or $CY^4$,

each of $Y^1$, $Y^2$ and $Y^3$, which may be the same or different, represents a hydrogen atom; a halogen atom; a $C_1$-$C_6$ alkyl group; a $C_1$-$C_6$ alkoxy group; a nitro group; a cyano group; or a phenyl group, and

$Y^4$ represents a hydroxy group; a mercapto group; a nitro group; a halogen atom; a $C_1$-$C_6$ alkyl group; a $C_2$-$C_6$ alkenyl group; a $C_2$-$C_6$ alkynyl group; a $C_1$-$C_6$ alkoxy group; a $C_3$-$C_6$ alkenyloxy group; a $C_3$-$C_6$ alkynyloxy group; a halo-$C_1$-$C_6$ alkyl group; a halo-$C_2$-$C_6$ alkenyl group; a halo-$C_2$-$C_6$ alkynyl group; a halo-$C_1$-$C_6$ alkoxy group; a halo-$C_3$-$C_6$ alkenyloxy group; a halo-$C_3$-$C_6$ alkynyloxy group; a $C_1$-$C_6$ alkoxy-$C_1$-$C_6$ alkyl group; a $C_3$-$C_6$ alkenyloxy-$C_1$-$C_6$ alkyl group; a $C_3$-$C_6$ alkynyloxy-$C_1$-$C_6$ alkyl group; a cyano group; a formyl group; a carboxyl group; a ($C_1$-$C_6$ alkoxy)carbonyl group; a ($C_3$-$C_6$ alkenyloxy)carbonyl group; a ($C_3$-$C_6$ alkynyloxy)carbonyl group; a phenyl group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a phenoxy group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a phenylthio group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a benzyloxy group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a benzylthio group optionally substituted with at least one member selected from the group consisting of $C_1$-$C_6$ alkyl, $C_1$-$C_6$ alkoxy, $C_1$-$C_6$ haloalkyl, ($C_1$-$C_6$ alkoxy)carbonyl, nitro and cyano groups and halogen atoms; a group represented by the formula,

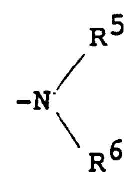

(in which each of $R^5$ and $R^6$ as described in Claim 1); a group represented by the formula,

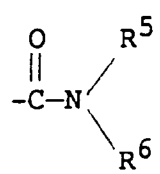

(in which $R^5$ and $R^6$ are as defined above); a group represented by the formula

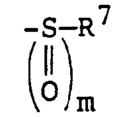

(in which $R^7$ and m are as described in Claim 1); a group represented by the formula

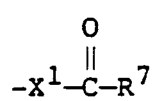

(in which $R^7$ is as defined above and $X^1$ is an oxygen atom or a sulfuratom; or a group represented by the formula,

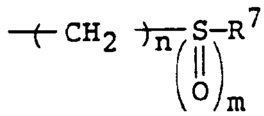

(in which $R^7$, m and n are as defined above).

3. A triazine derivative according to Claim 1 or 2, wherein each of $R^3$ and $R^4$, which may be the same or different, is an optionally substituted $C_1$-$C_6$alkoxy group.

4. A triazine derivative according to Claim 3, wherein both $R^3$ and $R^4$ are methoxy groups.

5. A triazine derivative according to any preceding claim wherein X is an oxygen atom.

6. A triazine derivative according to any preceding claim wherein Z is N or $CY^4$ in which $Y^4$ represents an optionally substituted phenyl group or a halogen atom.

7. A triazine derivative according to Claim 6, wherein Z is $CY^4$ in which $Y^4$ is an optionally substituted phenyl group.

8. A triazine derivative according to any preceding claim wherein each of $Y^1$, $Y^2$ and $Y^3$ is a hydrogen atom or a fluorine atom.

9. A triazine derivative according to Claim 1 selected from the following compound group:
4,6-dimethoxy-2-{3-chloro-2-(N,N-dimethylaminooxycarbonyl)phenoxy}-1,3,5-triazine
4,6-dimethoxy-2-{3-fluoro-2-(N,N-dimethylaminooxycarbonyl)phenoxy}-1,3,5-triazine
4,6-dimethoxy-2-{3-phenyl-2-(N,N-dimethylaminooxycarbonyl)phenoxy}-1,3,5-triazine.

10. A method for producing a triazine derivative according to Claim 1 which comprises reacting a phenol or thiophenol derivative represented by the formula,

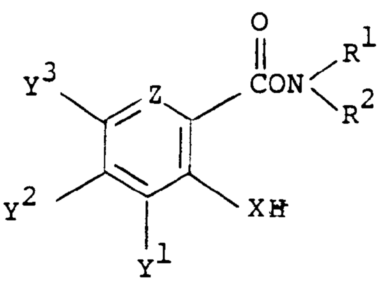

(II)

wherein $R^1$, $R^2$, $Y^1$, $Y^2$, $Y^3$, X and Z are each as defined in Claim 1, with a triazine derivative represented by the formula,

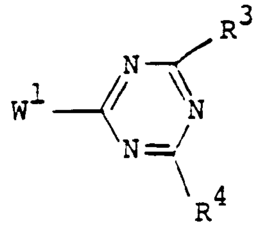

wherein $R^3$ and $R^4$ are each as defined in Claim 1 and W represents a halogen atom.